# EUROPÄISCHE PATENTSCHRIFT

(11) **EP 0 678 091 B1**
(45) Veröffentlichungstag und Bekanntmachung des Hinweises auf die Patenterteilung: **06.11.1996**
(21) Anmeldenummer: 94903867.3
(22) Anmeldetag: 23.12.1993
(51) Int. Cl.: C07D 309/08

(54) **VERFAHREN ZUR HERSTELLUNG VON TETRAHYDROPYRAN-4-CARBONSÄURE UND DEREN ESTERN**
METHOD OF PREPARING TETRAHYDROPYRAN-4-CARBOXYLIC ACID AND ITS ESTERS
PROCEDE PERMETTANT DE PREPARER DE L'ACIDE TETRAHYDROPYRANE-4-CARBOXYLIQUE ET SES ESTERS

(30) Priorität: 09.01.1993 DE 4300419
(43) Veröffentlichungstag der Anmeldung: 25.10.1995
(73) Patentinhaber: BASF AKTIENGESELLSCHAFT, 67056 Ludwigshafen (DE)
(72) Erfinder: FISCHER, Rolf, D-69121 Heidelberg (DE); SCHNURR, Werner, D-67273 Herxheim (DE); GOETZ, Norbert, D-67547 Worms (DE); KUEKENHOEHNER, Thomas, D-67459 Boehl-Iggelheim (DE)
(86) Internationale Anmeldenummer: EP9303670
(87) Internationale Veröffentlichungsnummer: WO9415931

(56) Entgegenhaltungen:
- EP-A- 0 284 969

## Beschreibung

Die vorliegende Erfindung betrifft ein Verfahren zur Herstellung von Tetrahydropyran-4-carbonsäure und deren Estern durch Umsetzung von 2,7-Dioxaspiro[4,4]nonan-1,6-dion mit Wasser oder Alkoholen in Gegenwart von sauer wirkenden Katalysatoren bei erhöhten Temperaturen.

Aus J. Chem. Soc. 1930, Seiten 2525 bis 2530 ist ein Verfahren zur Herstellung von Tetrahydropyran-4-carbonsäureestern bekannt, bei dem man β,β'-Dichlordiethylether mit dem Natriumsalz des Malonsäurediethylesters zu Tetrahydropyran-4,4-dicarbonsäurediethylester umsetzt, diesen über Tetrahydropyran-4,4-dicarbonsäure in Tetrahydropyran-4-carbonsäure überführt und schließlich zum gewünschten Tetrahydropyran-4-carbonsäureester verestert.

Aus EP-A-284 963 ist weiterhin bekannt, Tetrahydropyran-4-carbonsäureester durch Umsetzung von 3-(2-Hydroxyethyl)-butyrolacton oder deren Ester und Ether mit Alkoholen in Gegenwart von sauer wirkenden Katalysatoren, herzustellen.

Nachteilig an den bekannten Verfahren ist, daß sie entweder in zahlreichen Reaktionsschritten und unter Salzanfall oder nur mit niedrigen Ausbeuten verlaufen.

Der vorliegenden Erfindung lag daher die Aufgabe zugrunde, die Verbindungen I besser zugänglich zu machen.

Demgemäß wurde ein neues und verbessertes Verfahren zur Herstellung von Tetrahydropyran-4-carbonsäure und deren Estern der Formel I 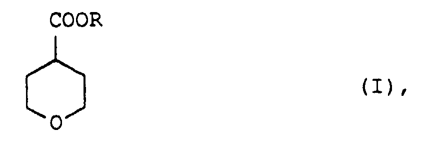 in der R Wasserstoff, C₁-C₈-Alkyl, C₃-C₈-Cycloalkyl, Aryl, C₇-C₁₂-Alkylphenyl und C₇-C₁₂-Phenylalkyl bedeutet, gefunden, welches dadurch gekennzeichnet ist, daß man 2,7-Dioxaspiro[4,4]-nonan-1,6-dion der Formel II 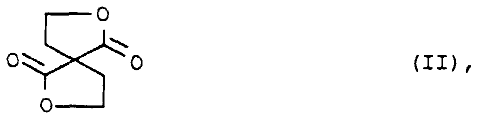 mit Wasser oder Alkoholen der Formel III

R-OH (III),

in Gegenwart von sauren Katalysatoren bei Temperaturen von 150 bis 350°C und Drücken von 0,01 bis 100 bar umsetzt.

Das 2,7-Dioxaspiro[4,4]nonan-1,6-dion II kann bevorzugt in geschmolzener (Smp.: 108 bis 109°C) oder gelöster Form, zusammen mit dem Alkohol III oder Wasser und gegebenenfalls einem Inertgas wie Stickstoff, Kohlendioxid oder Argon zu Tetrahydropyran-4-carbonsäure und deren Estern I umgesetzt werden.

Besonders vorteilhaft sind Reaktionstemperaturen von 200 bis 300°C, insbesondere von 220 bis 270°C und ein Druck von 0,1 bis 5 bar.

Die Umsetzung kann diskontinuierlich oder kontinuierlich vorgenommen werden, z.B. als Festbettreaktion mit Festbettkatalysatoren, beispielsweise in Sumpf- oder Rieselfahrweise in der Flüssig- oder Gasphase, z.B. in fester Form im Wirbelbett oder mit in der Flüssigphase suspendierten Festbett-Katalysatoren oder Homogenkatalysatoren.

Der Reaktionsaustrag kann mittels geeigneter Kühlvorrichtung kondensiert und anschließend durch fraktionierende Destillation aufgearbeitet werden. Die Tetrahydropyran-4-carbonsäure und deren Ester I können abgetrennt und nicht umgesetztes 2,7-Dioxaspiro[4,4]nonan-1,6-dion II bzw. die im Reaktionsaustrag gegebenenfalls enthaltenen Ether oder Ester des 3-(2'-Hydroxyethyl)-dihydro-2(3H)furanons, z.B. 3-(2'-Methoxyethyl)-dihydro-(2(3H)furanon, zurückgeführt werden (vgl. EP-A-284 963).

Die Umsetzung in der Flüssigphase kann beispielsweise so durchgeführt werden, daß man ein Gemisch aus 2,7-Dioxaspiro[4,4]-nonan-1,6-dion II und dem jeweiligen Alkohol III (bzw. Wasser) in Gegenwart eines suspendierten Festbettkatalysators oder eines homogen gelösten Katalysators auf die gewünschte Reaktionstemperatur erhitzt. Nach Ablauf der notwendigen Reaktionszeit wird das Reaktionsgemisch abgekühlt und der Katalysator, z.B. durch Filtration oder Neutralisation, entfernt. Das Reaktionsgemisch kann anschließend zur Gewinnung der gewünschten Tetrahydropyran-4-carbonsäureester fraktionierend destilliert werden.

Das Molverhältnis von Alkohol III bzw. Wasser zum 2,7-Dioxaspiro[4,4]-nonan-1,6-dion II beträgt üblicherweise 0,5 : 1 bis 50 : 1, bevorzugt 1 : 1 bis 30 : 1, besonders bevorzugt 2 : 1 bis 20 : 1.

Bei heterogenen Katalysatoren arbeitet man dabei mit Katalysatorbelastungen von 0,1 bis 10, insbesondere von 0,1 bis 5 g Lacton II pro g Katalysator und Stunde.

Geeignete Alkohole der allgemeinen Formel III sind beispielsweise Methanol, Ethanol, n-Propanol, iso-Propanol, tert.-Butanol, n-Butanol, iso-Butanol, sec.-Butanol, n-Pentanol, n-Hexanol, Phenol, Cyclopentanol, Cyclohexanol. Besonders geeignet sind Methanol, Ethanol, n-Propanol und iso-Propanol.

Als saure Katalysatoren eignen sich saure Homogenkatalysatoren, in der Flüssigphasenreaktion z.B. Mineralsäuren wie Schwefelsäure, Phosphorsäure, Salzsäure, Salpetersäure, Bromwasserstoffsäure oder Sulfonsäuren wie Benzolsulfonsäure und p-Toluolsulfonsäure, vorzugsweise jedoch Heterogenkatalysatoren, z.B. sauer wirkende Oxide von Elementen der III. und IV. Hauptgruppe sowie der IV. bis VI. Nebengruppe des Periodensystems der Elemente. Beispielsweise seien als saure Heterogenkatalysatoren Siliciumdioxid in Form von Kieselgel, Kieselgur, Quarz, Titandioxid, Zirkondioxid, Phosphorpentoxid, Vanadiumpentoxid, Bortrioxid, Aluminiumoxid, Chromoxide, Molybdänoxide, Wolframoxide, Zeolithe wie die Y-Zeolithe oder deren Gemische genannt. Besonders bevorzugt ist Aluminiumoxid.

Das Molverhältnis von saurem Homogenkatalysator zum 2,7-Dioxaspiro[4,4]nonan-1,6-dion II beträgt normalerweise 0,001 : 1 bis 1 : 1, bevorzugt 0,01 : 1 bis 0,1 : 1.

Das 2,7-Dioxaspiro[4,4]nonan-1,6-dion II läßt sich beispielsweise nach J. Chem. Soc., Perkin I, S. 521-530 (1977) durch Umsetzung von Malonsäurediethylester mit Ethylencarbonat in Gegenwart von Natriumiodid, nach J. Org. Chem. Bd. 28, S. 2809-2811 (1963) durch Hydrolyse von 1,5-Dibrom-3,3-dicyanopentan mit Schwefelsäure oder nach J. Org. Chem. Bd. 50, 1026-1031 (1985) durch Umsetzung von Malonsäure mit Ethylen in Gegenwart von Mangan(III)-acetat herstellen.

Der Substituent R in den Verbindungen I und III hat folgende Bedeutungen:
R
- C₁-C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, bevorzugt C₁- bis C₄-Alkyl wie Methyl, Ethyl, n-Propyl, iso-Propyl, n-Butyl, iso-Butyl, sec.-Butyl und tert.- Butyl, besonders bevorzugt Methyl, Ethyl, n-Propyl und iso-Propyl,
- C₃-C₈-Cycloalkyl wie Cyclopropyl, Cyclobutyl, Cyclopentyl, Cyclohexyl, Cycloheptyl und Cyclooctyl, bevorzugt C₅- bis C₈-Cycloalkyl wie Cyclopentyl, Cyclohexyl, Cycloheptyl und Cyclooctyl, besonders bevorzugt C₅- bis C₇-Cycloalkyl wie Cyclopentyl, Cyclohexyl und Cycloheptyl,
- Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
- C₇-C₁₂-Alkylphenyl wie 2-Methylphenyl, 3-Methylphenyl, 4-Methylphenyl, 2,4-Dimethylphenyl, 2, 5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl, bevorzugt 2- und 4-Methylphenyl,
- C₇-C₁₂-Phenylalkyl wie Benzyl, 1-Phenethyl, 2-Phenethyl, 1-Phenylpropyl, 2-Phenylpropyl, 3-Phenylpropyl, 1-Phenylbutyl, 2-Phenylbutyl, 3-Phenylbutyl und 4-Phenylbutyl, bevorzugt Benzyl, 1-Phenethyl und 2-Phenethyl.

Die nach dem erfindungsgemäßen Verfahren erhältlichen Tetrahydropyran- 4-carbonsäureester stellen wertvolle Zwischenprodukte dar, die z.B. zum Tetrahydropyran-4-carbaldehyd (vgl. DE-A-31 21 355, DE-A-33 14 816, DE-A-33 40 265, DE-A-38 21 197 und DE-A-40 39 918) weiterverarbeitet werden können.

### Herstellungsbeispiel

Über einen beheizten Tropftrichter (50°C). wurden pro Stunde 22 g einer 20 %igen Lösung von 2,7-Dioxaspiro[4.4]nonan-1,6-dion in Methanol in einen senkrecht stehenden Rohrreaktor getropft. Zusätzlich wurden 10 l/h Stickstoff zudosiert. Der Reaktor enthielt im oberen Teil Quarzringe und darunter 43 g Al₂O₃ als Katalysator und wurde bei 230°C betrieben. Der gasförmige Reaktionsaustrag wurde in Kühlfallen kondensiert und gaschromatographisch analysiert.

Dabei wurden 40 Mol-% Tetrahydropyran-4-carbonsäuremethylester, 33 Mol-% 3-(2-Methoxyethyl)-dihydro-2(3H)furanon (rückführbares Nebenprodukt) und 14 Mol-% 5-Oxaspiro[2.4]heptan-4-on gebildet. Die Tetrahydropyran-4-carbonsäuremethylester-Selektivität betrug demnach 60 %.

## Patentansprüche

1. Verfahren zur Herstellung von Tetrahydropyran-4-carbonsäure und deren Estern der Formel I 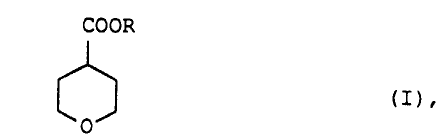 in der R Wasserstoff, C₁-C₈-Alkyl, C₃-C₈-Cycloalkyl, Aryl, C₇-C₁₂-Alkylphenyl und C₇-C₁₂-Phenylalkyl bedeutet,
dadurch gekennzeichnet, daß man 2,7-Dioxaspiro[4,4]-nonan-1,6-dion der Formel II 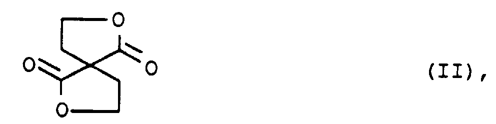 mit Wasser oder Alkoholen der Formel III
R-OH (III),
in Gegenwart von sauren Katalysatoren bei Temperaturen von 150 bis 350°C und Drücken von 0,01 bis 100 bar umsetzt.

2. Verfahren zur Herstellung von Tetrahydropyran-4-carbonsäure und deren Estern I nach Anspruch 1, dadurch gekennzeichnet, daß man die Umsetzung bei Temperaturen von 200 bis 300°C durchführt.

3. Verfahren zur Herstellung von Tetrahydropyran-4-carbonsäure und deren Estern I nach Anspruch 1, dadurch gekennzeichnet, daß man die Umsetzung bei Temperaturen von 220 bis 270°C durchführt .

4. Verfahren zur Herstellung von Tetrahydropyran-4-carbonsäure und deren Estern I nach Anspruch 1, dadurch gekennzeichnet, daß man als saure Katalysatoren sauer wirkende Oxide von Elementen der III. und IV. Hauptgruppe sowie der IV. bis VI. Nebengruppe des Periodensystems der Elemente verwendet.

5. Verfahren zur Herstellung von Tetrahydropyran-4-carbonsäure und deren Estern I nach Anspruch 1, dadurch gekennzeichnet, daß man als Katalysator Aluminiumoxid verwendet.

## Claims

1. A process for preparing tetrahydropyran-4-carboxylic acid and its esters of the formula I 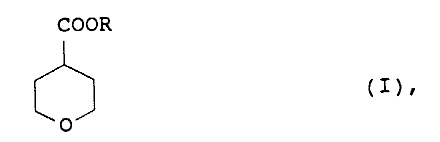 where R is hydrogen, C₁-C₈-alkyl, C₃-C₈-cycloalkyl, aryl, C₇-C₁₂-alkylphenyl or C₇-C₁₂-phenylalkyl, which comprises reacting 2,7-dioxaspiro[4.4]nonane-1,6-dione of the formula II 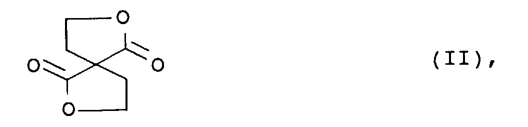 with water or alcohols of the formula III
R-OH (III),
in the presence of acidic catalysts at from 150 to 350°C and from 0.01 to 100 bar.

2. A process for preparing tetrahydropyran-4-carboxylic acid and its esters I as claimed in claim 1, wherein the reaction is carried out at from 200 to 300°C.

3. A process for preparing tetrahydropyran-4-carboxylic acid and its esters I as claimed in claim 1, wherein the reaction is carried out at from 220 to 270°C.

4. A process for preparing tetrahydropyran-4-carboxylic acid and its esters I as claimed in claim 1, wherein the acidic catalysts used are acidic oxides of elements of main groups III and IV and of subgroups IV to VI of the Periodic Table of the Elements.

5. A process for preparing tetrahydropyran-4-carboxylic acid and its esters I as claimed in claim 1, wherein the catalyst used is aluminum oxide.

## Revendications

1. Procédé de préparation d'acide tétrahydropyranne-4-carboxylique et de ses esters de formule I 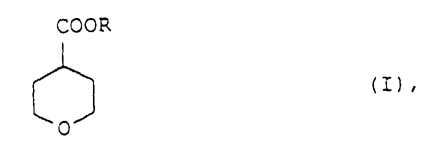 dans laquelle R représente un atome d'hydrogène ou un reste alkyle en C₁-C₈, cycloalkyle en C₃-C₈, aryle, alkylphényle en C₇-C₁₂ ou phénylalkyle en C₇-C₁₂, caractérisé en ce que l'on fait réagir de la 2,7-dioxaspiro[4,4]nonan-1,6-dione de formule II 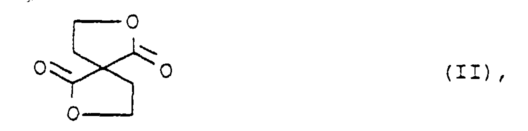 avec de l'eau ou des alcools de formule III
R-OH (III)
en présence de catalyseurs acides, à des températures de 150 à 350°C et sous des pressions de 0,01 à 100 bar.

2. Procédé de préparation d'acide tétrahydropyranne-4-carboxylique et de ses esters I selon la revendication 1, caractérisé en ce que l'on conduit la réaction à des températures de 200 à 300°C,

3. Procédé de préparation d'acide tétrahydropyranne-4-carboxylique et de ses esters I selon la revendication 1, caractérisé en ce que l'on conduit la réaction à des températures de 220 à 270°C.

4. Procédé de préparation d'acide tétrahydropyranne-4-carboxylique et de ses esters I selon la revendication 1, caractérisé en ce que l'on utilise, comme catalyseurs acides, des oxydes à action acide d'éléments des groupes IIIA et IVA, ainsi que des groupes IVB à VIB de la classification périodique.

5. Procédé de préparation d'acide tétrahydropyranne-4-carboxylique et de ses esters selon la revendication 1, caractérisé en ce que l'on utilise de l'oxyde d'aluminium comme catalyseur.
